Europäisches Patentamt

European Patent Office

Office européen des brevets

(19)

(11) **EP 1 238 094 B1**

(12) **EUROPÄISCHE PATENTSCHRIFT**

(45) Veröffentlichungstag und Bekanntmachung des Hinweises auf die Patenterteilung:
**04.02.2004   Patentblatt 2004/06**

(51) Int Cl.[7]: **C12P 13/00**
// C12N9:88

(21) Anmeldenummer: **00989884.2**

(22) Anmeldetag: **25.11.2000**

(86) Internationale Anmeldenummer:
**PCT/EP2000/011753**

(87) Internationale Veröffentlichungsnummer:
**WO 2001/044487 (21.06.2001 Gazette 2001/25)**

(54) **VERFAHREN ZUR HERSTELLUNG VON OPTISCH AKTIVEN CYANHYDRINEN UNTER VERWENDUNG VON R-OXYNITRILASE**

METHOD FOR THE PRODUCTION OF OPTICALLY ACTIVE CYANOHYDRINS USING R-OXYNITRILASE

PROCEDE DE PREPARATION DE CYANHYDRINES OPTIQUEMENT ACTIVES PAR UTILISATION D'OXYNITRILASE R

(84) Benannte Vertragsstaaten:
**AT BE CH DE DK ES FI FR GB IE IT LI NL SE**
Benannte Erstreckungsstaaten:
**SI**

(30) Priorität: **15.12.1999  AT  210899**

(43) Veröffentlichungstag der Anmeldung:
**11.09.2002   Patentblatt 2002/37**

(73) Patentinhaber: **DSM Fine Chemicals Austria Nfg GmbH & Co KG**
**4021 Linz (AT)**

(72) Erfinder:
• **PÖCHLAUER, Peter**
**A-4040 Linz (AT)**
• **WIRTH, Irma**
**A-4040 Linz (AT)**
• **MAYRHOFER, Herbert**
**A-4210 Engerwitzdorf (AT)**
• **NEUHOFER, Rudolf**
**A-4210 Mittertreffling (AT)**

(74) Vertreter: **Lindinger, Ingrid**
**DSM Fine Chemicals Austria Nfg GmbH & Co KG,**
**St. Peter-Strasse 25**
**4021 Linz (AT)**

(56) Entgegenhaltungen:
EP-A- 0 276 375          EP-A- 0 547 655
EP-A- 0 927 766          WO-A-99/63104
DE-A- 19 506 728         DE-A- 19 703 314
DE-C- 4 008 412          DE-C- 4 102 327

• EFFENBERGER F ET AL: "ENZYME-CATALYZED CYANOHYDRIN SYNTHESIS IN ORGANIC SOLVENTS" ANGEWANDTE CHEMIE INTERNATIONAL EDITION IN ENGLISH, Bd. 26, Nr. 5, 1987, Seiten 458-460, XP002165559 ISSN: 0570-0833 in der Anmeldung erwähnt
• OGNYANOV V I ET AL: "PREPARATION OF CHIRAL CYANOHYDRINS BY AN OXYNITRILASE-MEDIATED TRANSCYANATION" JOURNAL OF THE AMERICAN CHEMICAL SOCIETY, Bd. 113, Nr. 18, 1991, Seiten 6992-6996, XP002165560 ISSN: 0002-7863 in der Anmeldung erwähnt

EP 1 238 094 B1

**Beschreibung**

Cyanhydrine sind etwa zur Synthese von alpha-Hydroxysäuren, alpha-Hydroxyketonen, beta-Aminoalkoholen, die zur Gewinnung biologisch wirksamer Stoffe, z. B. pharmazeutischer Wirkstoffe, Vitamine oder auch pyrethroider Verbindungen Verwendung finden, von Bedeutung.

Die Herstellung eines Cyanhydrins kann durch Anlagerung von Blausäure (HCN) an die Carbonylgruppe eines Aldehyds oder eines unsymmetrischen Ketons erfolgen, wobei Enantiomerengemische unsymmetrischer Cyanhydrine entstehen.

Da in einem biologisch wirksamen Enantiomerengemisch normalerweise nur eines der beiden Enantiomere biologisch wirksam ist, hat es nicht an Versuchen gefehlt, ein Verfahren zur Herstellung des (R)-Enantiomeren eines optisch aktiven Cyanhydrins in möglichst hoher optischer Reinheit zu finden.

Viele Verfahren beruhen darauf, die Anlagerung von HCN an die Carbonylgruppe in Gegenwart eines chiralen Katalysators, beispielsweise einer Oxynitrilase durchzuführen.

Die Enantiomerenreinheit des herzustellenden Cyanhydrins hängt dabei in hohem Maße davon ab, wie stark die chemische Konkurrenzreaktion und Racemisierung unterdrückt werden können.

Wie aus J. Am. Chem. Soc. 1991, 113, S. 6992-6996 bekannt ist, ist es besonders bei Verfahren, die im wässrigen System arbeiten, schwierig auf Grund dieser Konkurrenzreaktion eine hohe Enantioselektivität und Enantiomerenreinheit zu erzielen.

Ein Weg, die chemische Konkurrenzreaktion und Racemisierung zu unterdrücken, wird in EP-A-0 326 063 angegeben, nach dem optisch aktive (R)-Cyanhydrine durch Umsetzung von aliphatischen, aromatischen oder heteroaromatischen Aldehyden oder Ketonen in wässrigem Milieu mit Blausäure in Gegenwart von (R)-Oxynitrilase (EC 4.1.2.10) aus Prunus amygdalus erhalten werden sollen, indem unter derart sauren Bedingungen, insbesondere bei $pH \leq 4,5$ und bei solchen Temperaturen gearbeitet wird, dass die chemische Konkurrenzreaktion und Racemisierung gegenüber der enzymatischen Synthese vernachlässigbar sind. Auf die unter diesen Bedingungen erhöhten Aktivitätsverluste des Biokatalysators wird dabei hingewiesen und die Beispiele lassen eine Favorisierung niedriger Temperaturen im Bereich von 5 bis 8 °C erkennen.

Da die Enzyme wasserlösliche Proteine und die Substrate hingegen nur schlecht wasserlösliche Verbindungen sind, wurde die Verwendung von mit Wasser mischbaren organischen Lösungsmitteln zur Verbesserung der Löslichkeit des Substrates und des Produktes vorgeschlagen.

So wurde zum Beispiel von Effenberger et al. (Angew. Chem. 99 (1987) S.491-492) die enzymatische Cyanhydrinbildung in wässrig-alkoholischen Systemen unter Variation des pH-Wertes, der Temperatur und Konzentration in Hinblick auf eine optimale Unterdrückung der Konkurrenzreaktionen untersucht. Die stereochemische Reinheit der gewünschten Endprodukte war dabei jedoch oft nicht zufriedenstellend. Als Verbesserung wurde vorgeschlagen, die enzymatische Umsetzung von Oxoverbindungen mit Blausäure in organischen mit Wasser nicht mischbaren Lösungsmitteln durchzuführen um die chemische Reaktion zu unterdrücken. Dabei wurde bevorzugt mit Ethylacetat und unter Verwendung von trägerfixierter (R)-Oxynitrilase gearbeitet. Auf diese Weise wurden zwar Produkte mit hoher optischer Reinheit erhalten, jedoch wurde durch die Immobilisierung des Enzymes ein beträchtlicher Verlust an Enzymaktivität beobachtet. Weiters wurde festgestellt, dass die nicht enzymatische Reaktion, die in der wässrigen Phase durch Addition der Blausäure an die Ausgangsverbindung zu racemischen Cyanhydrinen führt, eine ungewollte Abnahme der Enantiomerenreinheit des Produktes verursacht.

In J. Am. Chem. Soc. 1991, 113, S. 6992-6996 werden die Probleme, die mit der Verwendung von freier Blausäure zusammenhängen, durch eine Transcyanierung mittels Hydroxynitrilase unter Verwendung von Acetoncyanhydrin in einem zweiphasigen Reaktionsgemisch, bestehend aus einer wässrigen Pufferlösung und einem mit Wasser nicht mischbaren Lösungsmittel, umgangen. Der Nachteil dabei ist, dass das Volumen an organischem Lösungsmittel und dadurch des gesamten Reaktionsgemisches ziemlich groß ist in Bezug auf die eingesetzte Aldehydmenge. Weiters wird eine extrem lange Reaktionszeit und eine große Menge an Enzym benötigt. Letztendlich ist auch die optische Reinheit der Cyanhydrine zumeist nicht ausreichend für die enantiospezifische Synthese der Zielprodukte.

Als Verbesserung wird in EP-A1-0 547 655 ein Verfahren vorgeschlagen bei welchem optisch aktive Cyanhydrine aus Aldehyden oder Ketonen und Blausäure in einem Zweiphasensystem, bestehend aus einer homogenen wässrigen Lösung der Hydroxynitrilase und einem geeigneten organischen Lösungsmittel, das zumindest im mit Wasser Wesentlichen unmischbar ist, hergestellt werden, wobei die wässrige Lösung mit einem Acetatpuffer in einer Konzentration von 0,005 bis 0,1 mol pro Liter gepuffert wird und das Verhältnis von organischer Phase zu wässriger Phase zwischen 5:1 und 1:5 liegt. Das Reaktionssystem wird während der enzymatischen Umsetzung gerührt, wobei das Zweiphasensystem erhalten bleibt

Trotz der Reaktion bei einem pH-Wert von etwa 4,5 kann die chemische Reaktion jedoch auch in diesem Zweiphasensystem aus organischer Substratlösung und wässriger Enzymlösung nicht vollständig unterdrückt werden. Nachteilig bei diesem Verfahren ist, dass die ee-Werte der Cyanhydrine nur durch den Einsatz großer Enzymmengen verbessert werden konnte.

In EP 0 927 766 ist ein Verfahren zur Herstellung von (S)-Cyanhydrinen beschrieben. Dabei werden Aldehyde oder Ketone unter Verwendung einer (S)-Hydroxynitrillyase in einer Emulsion zu den entsprechenden (S)-Cyanhydrinen umgesetzt. (S)-Hydroxynitrillyase und (R)-Hydroxynitrillyasen unterscheiden sich, wie beispielsweise in Angew. Chem. 1994, 106, S.1612 beschrieben, signifikant sowohl in ihrer Struktur als auch in ihren Katalysatoreigenschaften.

Aufgabe der Erfindung war es ein verbessertes Verfahren zur Herstellung von optisch aktiven Cyanhydrinen zu finden, das eine hohe Enantiomerenreinheit bei gleichzeitig niedrigen Enzym- und Zeitbedarf gewährleistet

Es wurde nun unerwarteterweise gefunden, dass die Umsetzung einer Vielzahl von Carbonylverbindungen, wie etwa aliphatische, alicyclische, ungesättigte, aromatisch substituierte aliphatische, aromatische, sowie heteroaromatische Aldehyde und Ketone, zu den entsprechenden Cyanhydrinen mit hoher Ausbeute und hoher optischer Reinheit in gegenüber dem Stand der Technik konzentrierterer Fahrweise, mit geringerem Enzymeinsatz und in mit kürzeren Reaktionszeiten möglich ist, wenn die Reaktion in einer Emulsion durchgeführt wird. Unerwarteterweise bleibt die Enzymaktivität unter den Bedingungen einer Emulsion, die bei vielen Proteinen zur Desaktivierung führen, wie etwa bei hoher Rührenergie, stabil.

Gegenstand der vorliegenden Erfindung ist daher ein Verfahren zur Herstellung der (R)-Enantiomeren von optisch aktiven Cyanhydrinen durch Umsetzung eines Aldehyds oder eines Ketons mit einem Cyanidgruppendonor in Anwesenheit einer (R)-Oxynitrilase, das dadurch gekennzeichnet ist, dass ein Reaktionsgemisch aus

a) einem in einem organischen, mit Wasser nicht oder geringfügig mischbaren Verdünnungsmittel gelösten Aldehyd oder Keton,
b) einer wässrigen (R)-Oxynitrifaselösung und
c) einem Cyanidgruppendonor

mit einer Rührenergie über 500W/m$^3$ gerührt wird, sodass eine Emulsion entsteht, die bis zum Ende der enzymatischen Reaktion aufrecht bleibt, worauf nach beendeter enzymatischen Reaktion das entsprechende (R)-Cyanhydrin aus dem Reaktionsgemisch isoliert wird.

Als Ausgangsmaterialien im erfindungsgemäßen Verfahren werden ein Aldehyd oder ein Keton, ein Cyanidgruppendonor, eine wässrige Lösung einer (R)-Oxynitrilase und ein organisches, mit Wasser nicht oder geringfügig mischbares Verdünnungsmittel eingesetzt.

Unter Aldehyden sind dabei aliphatische, aromatische oder heteroaromatische Aldehyde zu verstehen. Unter aliphatischen Aldehyden sind dabei gesättigte oder ungesättigte, aliphatische, geradkettige, verzweigte oder cyclische Aldehyde zu verstehen. Bevorzugte aliphatische Aldehyde sind geradkettige Aldehyde mit insbesondere 2 bis 30 C-Atomen, bevorzugt von 2 bis 18 C-Atomen, die gesättigt oder ein- oder mehrfach ungesättigt sind. Der Aldehyd kann dabei sowohl C-C-Doppelbindungen als auch C-C-Dreifachbindungen aufweisen. Die aliphatischen, aromatischen oder heteroaromatischen Aldehyde können weiters unsubstituiert oder durch unter den Reaktionsbedingungen inerte Gruppen, beispielsweise durch gegebenenfalls substituierte Aryl- oder Heteroarylgruppen, wie Phenyl-, Phenoxy- oder Indolylgruppen, durch Halogen-, Hydroxy-, Hydroxy-$C_1$-$C_5$-Alkyl, $C_1$-$C_5$-Alkoxy-, $C_1$-$C_5$-Alkylthio-, Ether-, Alkohol-, Carbonsäureester-, Nitro- oder Azidogruppen substituiert sein.

Beispiele für aromatische oder heteroaromatische Aldehyde sind Benzaldezyd bzw. verschieden substituierte Benzaldehyde wie etwa 3,4-Difluorbenzaldehyd, 3-Phenoxybenzaldehyd, 4-Fluor-3-phenoxybenzaldehyd, Hydroxybenzaldehyd, Methoxybenzaldehyd, weiters Furfural, Methylfurfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd oder Pyridinaldehyde, Thienylaldehyde u.s.w..

Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Inolylgruppen, durch Halogen-, Ether-, Alkohol-, Carbonsäureester-, Nitro- oder Azidogruppen substituiert sein.

Beispiele für aromatische oder heteroaromatische Ketone sind Acetophenon, Indolylaceton u.s.w..

Aldehyde und Ketone, die sich für das erfindungsgemäße Verfahren eignen, sind bekannt oder wie üblich herstellbar.

Als Cyanidgruppendonor wird Blausäure zugesetzt. Die Blausäure kann dabei auch erst kurz vor der Reaktion aus einem ihrer Salze wie etwa NaCN oder KCN freigesetzt und in Substanz oder in gelöster Form dem Reaktionsgemisch zugegeben werden.

Als Oxynitrilase kommen (R)-Oxynitrilasen z.B. aus Prunus amygdalus, Prunus laurocerasus oder Prunus serotina, in Frage. Bevorzugt wird als Oxynitrilase (R)-Oxynitrilase aus Prunus amygdalus verwendet.

Das Enzym zeichnet sich durch eine hohe Beständigkeit gegenüber Lösungsmitteln aus. Daher besteht die Möglichkeit, für die enzymatische Reaktion verschiedene organische Solventien einzusetzen, die die Bildung einer Emulsion erlauben, was sich günstig auf die Produktivität des jeweiligen Prozesses auswirkt.

Die Oxynitrilase kann gereinigt oder ungereinigt, als solche oder immobilisiert eingesetzt werden.

Als organische Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden.

Beispiele dafür sind Diethylether, Diisopropylether Dibutylether, Methyl- tert. Butylether, Ethylacetat, Propylacetat, Toluol, Xylol, Cyclohexan, Trichlorethan, Chlorbenzol u.s.w.

Bevorzugt werden Methyl- tert. Butylether (MTBE), Diisopropyiether, Dibutylether und Ethylacetat oder ein Gemisch aus MTBE und Toluol eingesetzt.

Pro g Aldehyd oder Keton werden etwa 0,2 bis 20 g Verdünnungsmittel und 10 bis 2 000 IU Aktivität Oxynitrilase, bevorzugt etwa 50 bis 1000 IU, zugesetzt.

Ein IU (International Unit) bezeichnet diejenige Menge eines Enzympräparates, die die Bildung von einem Mikromol Produkt pro Minute katalysiert. Die benötigte Menge der jeweiligen Oxynitrilase ermittelt man am besten in einem Aktivitätstest, etwa gemäß dem Aktivitätstest der Fa. Sigma, analog Jorns et al. J. Biolog. Chem. 254, S. 12145-12152, 1979.

Pro Mol eingesetzte Aldehyd- oder Ketogruppe werden mindestens 1 Mol, bevorzugt 1 bis 5 Mole, besonders bevorzugt 1 bis 2 Mole, Cyanidgruppendonor zugegeben. Bei dem erfindungsgemäßen Verfahren liegt der Aldehyd oder das Keton gelöst in dem organischen Verdünnungsmittel vor. Zu dieser Lösung wird das Enzym in Form einer wässrigen Pufferlösung zugesetzt. Der pH-Wert dieser Lösung soll dabei unter 7, bevorzugt zwischen 3 und 6,5, liegen.

Das so erhaltene Reaktionsgemisch wird bei Temperaturen von 0° bis etwa 30°C, bevorzugt 5 bis 25°C, derart gerührt, daß eine Emulsion entsteht. Die dafür notwendige Rührerdrehzahl (N) hängt dabei von der sogenannten Vermögenskennzahl des eingesetzten Rührers (Po), dessen Durchmesser (d), dem Reaktionsvolumen (V) sowie der Dichte ($\rho$) des Reaktionsmediums ab. Aus diesen Faktoren läßt sich die Rührenergie (P/V), d. h. die Rührerleistung pro Reaktionsvolumen (Volumen des Reaktionsgemisches, nicht der Apparatur) ableiten.

$$P/V = \frac{P_o \cdot \rho \cdot N^3 \cdot d^5}{V}$$

Bevorzugt liegt die Rührenergie bei dem erfindungsgemäßen Verfahren über 500 W/m$^3$, besonders bevorzugt über 1000 W/m$^3$. Vergleichsweise werden bei bisher bekannten Verfahren, die im wässrigen, organischen oder zweiphasigen System arbeiten, etwa gemäß EP-A1-0 547 655, lediglich Rührenergien von ungefähr 100 W/m$^3$ erzielt.

Liegt das Reaktionsgemisch als Emulsion vor, so wird der Cyanidgruppendonor zugesetzt. Die Emulsion bleibt bis zum Reaktionsende aufrecht. Der Reaktionsverlauf kann dabei beispielsweise photometrisch über die Abnahme des Aldehyd- bzw. Ketongehaltes verfolgt werden.

Je nach Edukt wird bei der Wellenlänge gemessen, bei der das Edukt absorbiert und das entstehende Cyanhydrin nicht. Die Absorption des Reaktionsgemisches sinkt somit mit steigender Conversion proportional.

Es können jedoch auch zuerst alle Komponenten zusammengemischt und anschließend das so erhaltene Reaktionsgemisch derart gerühren werden, dass eine Emulsion erhalten wird.

Bei Verwendung eines Salzes der Blausäure kann zuerst die Blausäure aus einer Lösung des Salzes durch Zugabe von beispielsweise H$_2$SO$_4$ oder H$_3$PO$_4$ freigesetzt werden. Der pH-Wert dieser Lösung von Blausäure sollte unter 7, bevorzugt zwischen 4 und 6,5, liegen.

Anschließend werden zu der Blausäurelösung die wässrige Enzymlösung, das organische Verdünnungsmittel und der Aldehyd oder das Keton zugegeben, die Reaktion gestartet und gegebenenfalls der pH-Wert nachjustiert.

Auch hier ist wieder darauf zu achten, dass das Reaktionsgemisch derart gerührt wird, dass eine Emulsion entsteht, die wiederum bis zum Ende der Reaktion aufrecht bleibt.

Zur Aufarbeitung des Reaktionsgemisches und zur Isolierung des gebildeten Cyanhydrines werden übliche Techniken, welche zunächst die Emulsion brechen, wie etwa Filtration, Zentrifugation oder Koaleszierung eingesetzt. Anschließend werden die sich bildenden Phasen gegebenenfalls unter Zugabe von Demulgatoren aufgetrennt und die produkthaltige Phase aufgearbeitet.

Zur Gewinnung des entsprechenden Cyanhydrines werden dabei je nach Endprodukt bekannte Techniken wie Filtration, Destillation, Extraktion oder Kristallisation angewandt. Die so gewonnenen Cyanhydrine können gegebenenfalls durch Zugabe einer Säure vor der Weiterverarbeitung stabilisiert werden.

Beispiel 1: 2-Chlorbenzaldehyd

0,25 bis 1 ml R-OxynitrilaseLösung (E.C.4.1.2.10, 877 units/ml) wurden mit 50 mM Citrat/Phosphatpuffer (pH4) auf 4 ml verdünnt und der pH-Wert der Enzymlösung gegebenenfalls mit einigen Tropfen Citronensäurelösung auf pH 4 eingestellt. Zu dieser Lösung wurde eine Lösung von 3 ml t.-Butylmethylether und 0,8g (5,69 mmol) 2-Chlorbenzaldehyd zugegeben und anschließend 445 µl (11,38 mmol) Blausäure zugefügt. Die Reaktionsmischung wurde mittels Magnetrührer bei Raumtemperatur mit 500 und mit 900 rpm gerührt. Wurde mit 500 rpm gerührt (Vergleichsversuche), so lag ein Zweiphasensystem analog EP-A1-0 547 655 vor, während durch das Rühren mit 900 rpm eine Emulsion entstand.

Der Umsatz und die Enantiomerenreinheit des gebildeten (R)-Cyanhydrins wurde mittels GC analysiert. Dazu wurde eine Probe der Reaktionslösung zentrifugiert und 50 µl der organischen Phase mit Dichlormethan verdünnt. Nach einer Derivatisierung mit Acetylchlorid wurde auf einer Cyclodextrinsäule gaschromatografisch analysiert

Der Umsatz und die Enantiomerenreinheit in Abhängigkeit von der Enzymmenge und der Rührerdrehzahl (Zweiphasensystem bzw. Emulsion) sind aus den Tabellen 1 und 2 ersichtlich.

Tabelle 1:

| Zweiphasensystem (Vergleichsversuch) | | | | | |
|---|---|---|---|---|---|
| Zeit (h) | 0,25 ml Enzyml./500rpm | | 0,5 ml Enzyml./500rpm | | 1,0 ml Enzyml./500rpm | |
| | % Umsatz | %ee | % Umsatz | %ee | %Umsatz | %ee |
| 0 | 0 | | 0 | | 0 | |
| 0,5 | 21,6 | 68,2 | 16,8 | 70 | 25,5 | 83,7 |
| 1 | 39,2 | 68,6 | 53,8 | 76,5 | 56,3 | 85,5 |
| 1,5 | 52,2 | 68,1 | 67,6 | 76,5 | 85,9 | 86,4 |
| 2 | 54,3 | 67,7 | 78,4 | 76,5 | 85,6 | 86,5 |
| 3 | 51,5 | 66,6 | 84,2 | 76,4 | 93,8 | 86,4 |
| 3,5 | 71,6 | 66,3 | 86,6 | 76,3 | 96,8 | 86,3 |
| 4,5 | 78,2 | 65,8 | 92,3 | 76,4 | 98,7 | 86,0 |

Tabelle 2:

| Emulsion | | | | | |
|---|---|---|---|---|---|
| Zeit (h) | 0,25 ml Enzym./900rpm | | 0,5 ml Enzyml./900rpm | | 1,0 ml Enzyml./900rpm | |
| | % Umsatz | %ee | % Umsatz | %ee | %Umsatz | %ee |
| 0 | 0 | | 0 | | 0 | |
| 0,5 | | | | | | |
| 1 | | | | | | |
| 1,5 | 79,1 | 77,5 | 97,6 | 81,6 | 98,7 | 89,4 |
| 2 | | | | | | |
| 3 | 98 | 77,4 | 100 | 81,5 | 100 | 89,1 |
| 3,5 | | | | | | |
| 4,5 | | | | | | |

Beispiel 2: n-Butyraldehyd

1 ml R-Oxynitrilaselösung (E.C.4.1.2.10, 877 units/ml) wurden mit 50 mM Citrat/Phosphatpuffer (pH4) auf 4 ml verdünnt und der pH-Wert der Enzymlösung gegebenenfalls mit einigen Tropfen Citronensäurelösung auf pH 4 eingestellt. Zu dieser Lösung wurde eine Lösung von 3 ml t.-Butylmethylether und 0,8g (11 mmol) n-Butyraldehyd zugegeben und anschließend 860 µl (22 mmol) Blausäure zugefügt. Die Reaktionsmischung wurde mittels Magnetrührer bei Raumtemperatur mit 900 rpm gerührt, wodurch eine Emulsion entstand.

Der Umsatz und die Enantiomerenreinheit des gebildeten Cyanhydrins wurde mittels GC analysiert.

Dazu wurde eine Probe der Reaktionslösung zentrifugiert und 50 µl der organischen Phase mit Dichlormethan verdünnt Nach einer Derivatisierung mit Acetylchlorid wurde auf einer Cyclodextrinsäule gaschromatografisch analysiert.

Nach 5 Minuten war der Aldeyd vollständig zum entsprechenden (R)-Cyanhydrin mit einer Enantiomerenreinheit von 98% umgesetzt.

**Patentansprüche**

**1.** Verfahren zur Herstellung der (R)-Enantiomeren von optisch aktiven Cyanhydrinen durch Umsetzung eines Aldehyds oder eines Ketons mit einem Cyanidgruppendonor in Anwesenheit einer (R)-Oxynitrilase, **dadurch gekennzeichnet, dass** ein Reaktionsgemisch aus

a) einem in einem organischen, mit Wasser nicht oder geringfügig mischbaren Verdünnungsmittel gelösten Aldehyd oder Keton,
b) einer wässrigen (R)-Oxynitrilaselösung und
c) einem Cyanidgruppendonor

mit einer Rührenergie über 500W/m$^3$ gerührt wird, sodass eine Emulsion entsteht, die bis zum Ende der enzymatischen Reaktion aufrecht bleibt, worauf nach beendeter enzymatischen Reaktion das entsprechende (R)-Cyanhydrin aus dem Reaktionsgemisch isoliert wird.

**2.** Verfahren nach Anspruch 1, **dadurch gekennzeichnet, dass** ein aliphatischer, aromatischer oder heteroaromatischer Aldehyd oder ein unsymmetrisches Keton umgesetzt wird.

**3.** Verfahren nach Anspruch 1, **dadurch gekennzeichnet, dass** als Cyanidgruppendonor Blausäure zugesetzt wird.

**4.** Verfahren nach Anspruch 1, **dadurch gekennzeichnet, dass** als Oxynitrilase eine (R)-Oxynitrilase aus Prunus amygdalus eingesetzt wird.

**5.** Verfahren nach Anspruch 1, **dadurch gekennzeichnet, dass** als Verdünnungsmittel mit Wasser nicht oder geringfügig mischbare aliphatische oder aromatische Kohlenwasserstoffe, die gegebenenfalls halogeniert sind, Alkohole, Ether oder Ester oder Gemische verwendet werden.

**6.** Verfahren nach Anspruch 1, **dadurch gekennzeichnet, dass** als Verdünnungsmittel Methyl-tert. Butylether, Diisopropylether, Dibutylether, Ethylacetat oder ein Gemisch aus Methyl- tert. Butylether und Toluol verwendet wird.

**7.** Verfahren nach Anspruch 1, **dadurch gekennzeichnet, daß** die Reaktionstemperatur 0°C bis 30°C beträgt.

**8.** Verfahren nach Anspruch 1, **dadurch gekennzeichnet, dass** nach beendeter enzymatischen Reaktion zur Isolierung des entsprechenden (R)-Cyanhydrin aus dem Reaktionsgemisch zunächst die Emulsion durch Filtration, Zentrifugation oder Koaleszierung gebrochen und anschließend die sich bildenden Phasen gegebenenfalls unter Zugabe von Demulgatoren aufgetrennt und die produkthaltige Phase aufgearbeitet wird.

**9.** Verfahren nach Anspruch 8, **dadurch gekennzeichnet, dass** die Aufarbeitung der produkthaltigen Phase je nach Endprodukt durch Filtration, Destillation, Extraktion oder Kristallisation erfolgt.

**Claims**

**1.** Method for the production of (R)-enantiomers of optically active cyanohydrins by reacting an aldehyde or a ketone with a cyanide group donor in the presence of an (R)-oxynitrilase, **characterized in that** a reaction mixture of

a) an aldehyde or ketone dissolved in an organic, water-immiscible or only slightly water-miscible diluent,
b) an aqueous (R)-oxynitrilase solution and
c) a cyanide group donor

is stirred with a stirrer energy greater than 500 W/m$^3$, so that an emulsion forms which is maintained up to the end of the enzymatic reaction, whereupon, after the enzymatic reaction is terminated, the corresponding (R)-cyanohydrin is isolated from the reaction mixture.

**2.** Method according to Claim 1, **characterized in that** an aliphatic, aromatic or heteroaromatic aldehyde or an unsymmetrical ketone is reacted.

**3.** Method according to Claim 1, **characterized in that** the cyanide group donor added is hydrocyanic acid.

**4.** Method according to Claim 1, **characterized in that** the oxynitrilase used is an (R)-oxynitrilase from Prunus amygdalus.

**5.** Method according to Claim 1, **characterized in that** water-immiscible or only slightly water-miscible aliphatic or aromatic hydrocarbons which may be halogenated, alcohols, ethers or esters or mixtures are used as diluent.

**6.** Method according to Claim 1, **characterized in that** the diluent used is methyl tert-butyl ether, diisopropyl ether, dibutyl ether, ethyl acetate or a mixture of methyl tert-butyl ether and toluene.

**7.** Method according to claim 1, **characterized in that** the reaction temperature is 0°C to 30°C.

**8.** Method according to claim 1, **characterized in that** after the enzymatic reaction is completed, to isolate the corresponding (R)-cyanohydrin from the reaction mixture, the emulsion is first broken by filtration, centrifugation or coalescence and the phases forming are then separated, if appropriate with addition of demulsifiers, and the product-containing phase is worked up.

**9.** Method according to Claim 8, **characterized in that** the product-containing phase, depending on the end product, is worked up by filtration, distillation, extraction or crystallization.

**Revendications**

**1.** Procédé de préparation des énantiomères R de cyanhydrines optiquement actives par réaction d'un aldéhyde ou d'une cétone avec un donneur de radical cyanure en présence d'une (R)-oxynitrilase, qui est **caractérisé en ce que** le mélange réactionnel, consiste en :

    (a) un aldéhyde ou une cétone dissoute dans un agent de dilution organique non ou peu miscible à l'eau ;
    (b) une solution aqueuse de (R)-oxynitrilase, et
    (c) un donneur de radical cyanure,

avec une énergie d'agitation supérieure à 500 W/m$^3$, de sorte qu'il se forme une émulsion, qui persiste jusqu'à la fin de la réaction enzymatique, où on isole la (R)-cyanhydrine correspondante du mélange de réaction à la fin de la réaction enzymatique.

**2.** Procédé selon la revendication 1, **caractérisé en ce que** l'on fait réagir un aldéhyde aliphatique, aromatique ou hétéroaromatique ou une cétone asymétrique.

**3.** Procédé selon la revendication 1, **caractérisé en ce que** l'on ajoute l'acide cyanhydrique comme donneur de radical cyanure.

**4.** Procédé selon la revendication 1, **caractérisé en ce que** l'on met en oeuvre comme oxynitrilase, une (R)-oxynitrilase de *Prunus amygdalus*.

**5.** Procédé selon la revendication 1, **caractérisé en ce que** l'on utilise comme agent de dilution, un hydrocarbure aliphatique ou aromatique, non ou peu miscible à l'eau, qui est le cas échéant halogéné, un alcool, un éther ou un ester ou un mélange.

**6.** Procédé selon la revendication 1, **caractérisé en ce que** l'on utilise comme agent de dilution, le méthyl-t-butyléther, le butyléther, le diisopropyléther, le dibutyléther, l'acétate d'éthyle ou un mélange de méthyl-t-butyléther et de toluène.

**7.** Procédé selon la revendication 1, **caractérisé en ce que** la température de réaction se situe entre 0°C et 30°C.

8. Procédé selon la revendication 1, **caractérisé en ce que**, à la fin de la réaction enzymatique, pour isoler la (R)-cyanhydrine correspondante depuis le mélange réactionnel, on rompt d'abord l'émulsion par filtration, centrifugation ou coalescence et ensuite, on sépare les phases formées, le cas échéant par addition d'anti-émulsionnants et on traite la phase contenant le produit.

9. Procédé selon la revendication 8, **caractérisé en ce que** le traitement de la phase contenant le produit est réalisé selon le produit final, par filtration, distillation, extraction ou cristallisation.